# EUROPÄISCHE PATENTSCHRIFT

(11) **EP 2 311 366 B1**
(45) Veröffentlichungstag und Bekanntmachung des Hinweises auf die Patenterteilung: **18.06.2014**
(21) Anmeldenummer: 10013248.9
(22) Anmeldetag: 02.10.2010
(51) Int. Cl.: A61B 1/06, A61B 1/12, G02B 23/24, F21K 99/00, A61B 1/04, F21Y 101/02

(54) **LED-Beleuchtungsmodul**
LED lighting module
Module d'éclairage à DEL

(30) Priorität: 19.10.2009 DE 102009049683
(43) Veröffentlichungstag der Anmeldung: 20.04.2011
(73) Patentinhaber: Richard Wolf GmbH, 75438 Knittlingen (DE)
(72) Erfinder: Weber, Bernd Claus, Dr., 76228 Karlsruhe (DE); Heimberger, Rudolf, 75038 Oberderdingen (DE); Schrumpf, Klaus, 76703 Kraichtal-Münzesheim (DE); Mahlkow, Adrian, Dr., 13359 Berlin (DE)
(74) Vertreter: Hemmer, Arnd

(56) Entgegenhaltungen:
- EP-A1- 1 911 389
- WO-A2-02/33312
- JP-A- 2003 024 276

## Beschreibung

Die Erfindung betrifft ein endoskopisches Instrument mit einem LED-Beleuchtungsmodul.

Bei Endoskopen kann es von Vorteil sein, eine Beleuchtungseinrichtung in Form einer LED direkt im Bereich des distalen Endes des Endoskopes anzuordnen, da so auf Lichtleiter im Inneren des Endoskopes verzichtet werden kann. Bei der Verwendung von LED am distalen Ende des Endoskopes gibt es jedoch das Problem, die von der LED erzeugte Abwärme abzuführen. Dies ist erforderlich, um beispielsweise Verbrennungen von umgebendem Gewebe zu vermeiden. Beispielsweise aus JP 11-216113 A ist es bekannt, die LED mithilfe von zugeführter Luft zu kühlen.

Aus EP 1 911 389 A1 ist eine Beleuchtungseinrichtung für ein Endoskop bekannt, bei welchem die von der LED erzeugte Abwärme über Anschlusselektroden der LED abgeführt wird. Jedoch sind diese Anschlusselektroden im Querschnitt sehr groß und starr, so dass ein Einsatz in einem flexiblen Endoskop nur mit Einschränkungen möglich ist.

JP 2003/024276 offenbart ein Endoskop mit einer LED-Beleuchtungseinrichtung. Die elektrischen Anschlussleitungen der LED sind zur elektromagnetischen Abschirmung als Koaxialkabel ausgeführt.

Es ist Aufgabe der Erfindung, ein endoskopisches Instrument mit einem LED-Beleuchtungsmodul zu schaffen, welches eine flexible Ausbildung des Endoskopschaftes ermöglicht und gleichzeitig eine gezielte Abfuhr der von der LED erzeugten Wärme ermöglicht.

Diese Aufgabe wird durch ein endoskopisches Instrument mit zumindest einem LED-Beleuchtungsmodul mit den im Anspruch 1 angegebene Merkmalen gelöst. Bevorzugte Ausführungsformen ergeben sich aus den Unteransprüchen, der nachfolgenden Beschreibung sowie den beigefügten Figuren.

Das erfindungsgemäße endoskopische Instrument weist zumindest ein LED-Beleuchtungsmodul auf. Dieses ist so ausgebildet, dass die zumindest eine der Beleuchtung dienende LED im Bereich des distalen Endes des Instrumentes angeordnet ist. Auf diese Weise kann auf die üblicherweise verwendeten Lichtleiter im Endoskopschaft verzichtet werden. Lichtleiterbündel sind empfindlich und weisen häufig optische Verluste auf. Auch diese Nachteile werden durch die Anordnung einer LED am distalen Ende des Instrumentes ausgeräumt.

Das Beleuchtungsmodul weist zumindest eine LED auf, es können jedoch auch mehrere LED Verwendung finden, beispielsweise, um eine größere Beleuchtungsstärke zu erzielen. Die zumindest eine LED weist in bekannter Weise eine elektrische Anschlussleitung auf, über welche die LED mit Energie versorgt wird. Die elektrische Anschlussleitung würde in einem medizinischen Instrument bzw. Endoskop dann von der LED zum proximalen Ende des Instrumentes verlaufen und dort oder alternativ auch außerhalb des Instruments mit einer Stromquelle verbunden.

Erfindungsgemäß ist vorgesehen, die elektrische Anschlussleitung auch zur Abfuhr der von der LED erzeugten Abwärme zu verwenden. Das heißt, es werden die wärmeleitenden Eigenschaften der elektrischen Anschlussleitung, insbesondere deren metallische Leiter verwendet, um die Verlustwärme abzuführen. Dazu wird zumindest einer der elektrischen Leiter in der Anschlussleitung so dimensioniert, dass er eine ausreichende Wärmeleitung zulässt, insbesondere einen ausreichend großen Wärmeleitwert aufweist, um einen erheblichen Teil, vorzugsweise den Großteil der an der LED anfallenden Verlustwärme von der LED wegzuführen. Als elektrische Leiter werden bevorzugt Materialien mit einer hohen Wärmeleitfähigkeit, wie z. B. Silber oder Kupfer oder eine Legierung mit einem ausreichend hohen Anteil derartiger Materialien. Um eine ausreichende Wärmeübertragung, insbesondere einen ausreichend großen absoluten Wärmeleitwert des oder der elektrischen Leiter zu realisieren, muss gemeinsam mit der Wärmeleitfähigkeit darüber hinaus die Querschnittsfläche des Leiters optimiert werden. Bei größerer Wärmeleitfähigkeit kann ein kleinerer Querschnitt ausreichen. Bei kleinerer spezifischer Wärmeleitfähigkeit ist ein größerer Materialquerschnitt erforderlich. Da sich die elektrische Anschlussleitung üblicherweise in entgegengesetzter Richtung zu der Abstrahlrichtung des Lichtes erstreckt, kann somit über die elektrische Anschlussleitung die entstehende Wärme von dem beleuchteten Bereich weggeführt werden. Bei dem endoskopischen Instrument wird so die Wärme in Richtung des proximalen Endes des Instrumentes abgeführt und somit der den distalen Bereich des Instrumentes umgebende Bereich des zu beobachtenden Objektes, beispielsweise das umgebende Gewebe, idealerweise weitgehend frei gehalten von der von der LED erzeugten Abwärme.

Um eine optimierte Wärmeübertragung von der LED auf den zumindest einen Leiter der elektrischen Anschlussleitung zu gewährleisten, wird dieser wärmeleitend mit der LED verbunden, beispielsweise angelötet, wobei idealerweise ein möglichst großflächiger Verbindungs- bzw. Wörmeübergangsbereich geschaffen wird.

Erfindungsgemäß ist die Anschlussleitung als Koaxialkabel ausgebildet, und zumindest einer der elektrischen Leiter des Koaxialkabels ist direkt in wärmeleitender Verbindung mit der LED. Als Koaxialkabel ist dabei ein Kabel mit zumindest einem inneren elektrischen Leiter und zumindest einem diesen umgebenden äußeren elektrischen Leiter oder ein Kabel mit vergleichbarem Aufbau zu verstehen, wobei die Leiter nicht unbedingt konzentrisch zueinander angeordnet sein müssen. Durch die Verwendung eines Koaxialkabels oder einer vergleichbaren Kabelanordnung kann bei ausreichender Flexibilität des Kabels, welche insbesondere für biegbare Systeme, wie sie in biegbaren Endoskopen eingesetzt werden, wichtig ist, eine große Querschnittsfläche des oder der elektrischen Leiter bereitgestellt werden, welche zur Wärmeabfuhr genutzt werden können. In einem Koaxialkabel können bei gleichzeitig minimiertem Außendurchmesser maximale Querschnittsflächen für die Leiter, welche zur Wärmeabfuhr genutzt werden können, erreicht werden. Bei möglichst kleinem Außenumfang wird hier eine maximal große Querschnittsfläche und so eine maximal mögliche Wärmeabfuhr erreicht.

Wie ausgeführt ist das Koaxialkabel bevorzugt flexibel ausgebildet, so dass es im Schaft eines flexiblen Endoskopes eingesetzt werden kann und die Biegbarkeit des Endoskopschaftes nicht beeinträchtigt. Dazu kann weiter bevorzugt zumindest einer der Leiter des Koaxialkabels aus mehreren einzelnen elektrischen Leitern, insbesondere Drähten zusammengesetzt sein. Insbesondere kann ein solcher Leiter aus einer Vielzahl von Litzen zusammengesetzt sein. Diese können, wie es von herkömmlichen Koaxialkabeln her bekannt ist, miteinander verflochten sein oder einfach sich in Längsrichtung des Kabels erstreckend gebündelt in dem Kabel angeordnet sein, insbesondere der äußere Leiter kann als Drahtgeflecht ausgebildet sein. Die Verwendung einer Vielzahl von Litzen stellt eine große Flexibilität des Kabels sicher.

Bevorzugt werden mehrere oder gar alle elektrischen Leiter der Anschlussleitung zur Ableitung der von der LED erzeugten Abwärme verwendet. Dazu sind vorzugsweise mehrere oder alle elektrische Leiter der Anschlussleitung wärmeleitend mit der LED verbunden, d. h. beispielsweise verlötet. Dabei werden idealerweise für alle diese Leiter möglichst großflächige Wärmeübergangsbereiche zur LED hin geschaffen, so dass der Wärmeübergang der LED auf die Leiter optimiert wird.

Weiter bevorzugt weist der zumindest eine elektrische Leiter der Anschlussleitung, welcher zur Wärmeabfuhr genutzt wird, eine größere, vorzugsweise mindestens dreimal, weiter bevorzugt mindestens fünfmal größere Querschnittsfläche auf, als für die elektrische Leistungsübertragung zu der LED erforderlich ist. Durch diese gegenüber der für die elektrische Leistungsübertragung nötigen Querschnittfläche vergrößerte Querschnittsfläche wird eine ausreichende Wärmeabfuhr gewährleistet. So kann ein Großteil der von der LED erzeugten Abwärme über diesen elektrischen Leiter abgeführt werden. Weiter bevorzugt weisen mehrere oder alle elektrischen Leiter der Anschlussleitung eine derart vergrößerte Querschnittsfläche auf.

Der zumindest eine elektrische Leiter des Anschlusskabels ist vorzugsweise mit einer maximal großen Querschnittsfläche wärmeleitend mit der LED verbunden. Auf diese Weise wird ein optimaler Wärmeübergang von der LED auf den Leiter gewährleistet.

Der zumindest eine elektrische Leiter der Anschlussleitung ist direkt wärmeleitend mit der LED verbunden. Durch diese direkte Verbindung der Leuchtdiode mit dem zumindest einen Leiter des Anschlusskabels wird ein optimierter Wärmeübergang geschaffen. Die elektrischen Anschlusskontakte der Leuchtdiode können einen kleineren Querschnitt aufweisen als der Querschnitt des Leiters der Anschlussleitung. Um dennoch einen optimierten Wärmeübergang von der Leuchtdiode auf die gesamte Querschnittsfläche des Leiters der Anschlussleitung zu schaffen, können weitere Bereiche der LED, welche nicht dem elektrischen Anschluss dienen, in wärmeleitenden Kontakt mit dem Leiter der Anschlussleitung gebracht sein, beispielsweise über ein wärmeleitendes Medium wie Wärmeleitpaste oder Ähnliches. Dieses wärmeleitende Medium kann dabei elektrisch isolierend ausgebildet sein.

Die elektrische Anschlussleitung ist vorzugsweise an einer rückseitigen Fläche wärmeleitend mit der zumindest einen LED verbunden. Die rückseitige Fläche der LED ist dabei eine Fläche, welche der Abstrahlrichtung für das von der LED erzeugte Licht abgewandt ist. Bei Anordnung in einem endoskopischen Instrument ist dieses vorzugsweise die dem proximalen Ende des Instrumentes zugewandte rückseitige Fläche der LED.

Besonders bevorzugt wird zumindest der äußere elektrische Leiter der Anschlussleitung, welche als Koaxialkabel oder als vergleichbares Kabel ausgebildet ist, wärmeleitend mit der LED verbunden. Dadurch wird der Wärmefluss über die Mantelfläche der Anschlussleitung entlang dem Endoskopschaft an die äußere Umgebung optimiert. Es ist jedoch auch möglich, dass sowohl der äußere als auch der innere elektrische Leiter des Koaxialkabels wärmeleitend mit der LED verbunden sind, um der Wärmeabfuhr zu dienen.

Um die Querschnittsfläche des wärmeleitenden Leiters zu maximieren, kann der äußere Leiter des Koaxialkabels in radialer Richtung eine gröere Dicke als eine Isolationsschicht aufweisen, welche zwischen einem inneren und dem äußeren elektrischen Leiter des Koaxialkabels gelegen ist. Das heißt, der äußere elektrische Leiter des Koaxialkabels weist bei dieser Ausführungsform bevorzugt eine größere Querschnittsfläche auf, als es bei bekannten Koaxialkabeln üblich ist. Idealerweise wird nur eine sehr dünne Isolationsschicht zwischen dem inneren und dem äußeren Leiter vorgesehen, welche gerade die elektrische Isolation sicherstellen kann. Auf diese Weise wird in der Querschnittsfläche der schlecht wärmeleitende Materialanteil des Kabels minimiert, während die wärmeleitenden Teile, nämlich die elektrischen Leiter im Querschnitt möglichst groß ausgebildet werden können. Gleichzeitig kann der Außendurchmesser des Koaxialkabels möglichst klein gehalten werden, was im Hinblick auf die beengten Platzverhältnisse im Schaft eines ßendoskopischen Instrumentes von Vorteil ist.

Um die Querschnittsfläche der Isolationsschicht weiter zu minimieren, kann es bevorzugt sein, dass der innere Leiter des Koaxialkabels eine minimierte Querschnittsfläche aufweist, welche in ihrer Größe an die zu übertragende elektrische Leistung angepasst ist. Das heißt, bei dieser Ausführungsform wird die Querschnittsfläche des inneren elektrischen Leiters vorzugsweise nur so groß gewählt, wie es für die zu der LED zu übertragende elektrische Leistung erforderlich ist. So wird diese Querschnittsfläche und die Umfangslänge im Querschnitt möglichst klein gehalten, wodurch auch die umfängliche Erstreckung der erforderlichen Isolationsschicht und damit deren Querschnittsfläche minimiert wird. Stattdessen wird bei dieser Ausführungsform bevorzugt die Querschnittsfläche des äußeren Leiters ausreichend groß ausgebildet, um die erforderliche Wärmeabfuhr von der LED bewerkstelligen zu können. Gemäß einer weiteren bevorzugten Ausführungsform kann auf eine elektrische Isolation am Außenumfang des äußeren Leiters des Koaxialkabels verzichtet werden. Eine elektrische Isolation ist bei dieser Ausgestaltung nur zwischen den beiden konzentrischen Leitern des Koaxialkabels, welche zur elektrischen Energieversorgung der LED dienen, vorgesehen. Dadurch wird eine weitere Verbesserung des Verhältnisses von gut wärmeleitendem zu schlecht wärmeleitendem Anteil am Querschnitt der Anschlussleitung erzielt.

Ein elektrischer Kurzschluss zwischen den beiden konzentrischen Leitern wird durch die Isolation zwischen den elektrischen Leitern ausgeschlossen. Ein Kurzschluss über äußere Elemente des endoskopischen Instrumentes ist aufgrund der konzentrischen Anordnung ausgeschlossen. Auf diese Weise kann somit der Gesamtquerschnitt des Koaxialkabels optimal zur Energieübertragung und Wärmeleitung ausgenutzt werden.

Das Beleuchtungsmodul kann fest in das endoskopische Instrument integriert sein. Dabei kann das Beleuchtungsmodul als vorgefertigtes Modul bereitgestellt werden, welches dann in das Instrument integriert wird. Darüber hinaus ist es auch möglich, das Beleuchtungsmodul entnehmbar bzw. austauschbar in dem endoskopischen Instrument anzuordnen, so dass es beispielsweise bei Beschädigung leicht ausgetauscht oder zur Reinigung und Desinfektion entnommen werden kann. Darüber hinaus ist es auch möglich, unterschiedliche Beleuchtungsmodule für verschiedene Einsatzzwecke bereitzustellen, welche in dem Instrument ausgetauscht werden oder aber auch fest in dieses integriert sein können. Die elektrische Anschlussleitung erstreckt sich von der am distalen Ende des endoskopischen Instrumentes angeordneten LED zum proximalen Ende und stellt somit die elektrische Energieversorgung von einer Stromquelle zum distalen Ende des Instrumentes und der dort angeordneten LED sicher. Ferner dient sie, wie oben beschrieben, der Abfuhr der Verlust- bzw. Abwärme von der LED. Diese Wärme wird von der Anschlussleitung zumindest zum Teil zum proximalen Ende des Instrumentes abgeführt. Dadurch kann gegebenenfalls zumindest ein Teil der Abwärme der LED von der Anschlussleitung zu einem beabstandet zu der LED gelegenen Kühlkörper abgeführt werden, mit welchem die elektrische Anschlussleitung bzw. zumindest ein Leiter der elektrischen Anschlussleitung ebenfalls wärmeleitend verbunden ist. Über diesen Kühlkörper kann die Abwärme dann nach außen, beispielsweise an die Umgebungsluft abgestrahlt werden. Bei diesem Kühlkörper kann es sich in der einfachsten Ausführung um die Oberfläche des Endoskophandgriffes oder eine sonst bereits vorhandene Schnittstelle zur Umgebung handeln. Darüber hinaus findet auch eine gewisse Wärmeabgabe über die Außenumfangsfläche der elektrischen Anschlussleitung, insbesondere in Form eines Koaxialkabels statt, so dass so die Abwärme der LED über die gesamte Länge des Beleuchtungsmoduls bzw. dessen Anschlussleitung an die Umgebung abgegeben wird. Auf diese Weise wird auch an der (Endoskop-) Oberfläche die punktuell auftretende maximale Temperatur reduziert, da die Wärmeenergie insgesamt über eine größere Fläche verteilt abgegeben wird. Auf diese Weise können Beschädigungen bzw. Verbrennungen von Gewebe vermieden werden.

Zur Optimierung der Wärmeabfuhr ist die Anschlussleitung in dem endoskopischen Instrument vorzugsweise als durchgehende Leitung vom distalen zum proximalen Ende ausgebildet. So werden Verbindungsstellen, welche einen schlechteren Wärmeübergang aufweisen, vermieden und insgesamt die Wärmeleitfähigkeit über die gesamte Länge vom distalen zum proximalen Ende des Instrumentes optimiert.

Weiter bevorzugt ist die LED umfänglich und/oder frontseitig von Elementen umgeben, welche eine geringere Wärmeleitfähigkeit als die elektrische Anschlussleitung aufweisen. Diese Elemente können beispielsweise in Form einer thermischen Isolationsschicht, beispielsweise aus Kunststoff ausgebildet sein. Dadurch wird sichergestellt, dass der Großteil der Abwärme über die elektrische Anschlussleitung abgeführt wird und nicht an die direkt die LED umgebenden Bereiche, insbesondere frontseitig der LED abgegeben wird. Auf diese Weise können Temperaturspitzen, welche zu Verbrennungen führen könnten, im Bereich des distalen Endes des endoskopischen Instrumentes vermieden werden. Diese Elemente mit geringerer Wärmeleitfähigkeit, d. h. thermische isolierenden Elemente, können als Teil des Beleuchtungsmoduls direkt die LED umgeben oder aber auch das Beleuchtungsmodul umgebend beispielsweise in dem endoskopischen Instrument ausgebildet sein. So kann auch beispielsweise die Wandung der Spitze oder des Kopfes des endoskopischen Instrumentes aus Kunststoff und damit thermisch isolierend ausgebildet sein.

Nachfolgend wird die Erfindung beispielhaft anhand der beigefügten Figuren beschrieben. In diesen zeigt:
- Fig. 1: schematisch den Aufbau eines erfindungsgemäßen LED- Beleuchtungsmoduls,
- Fig. 2: schematisch die Einbindung eines LED- Beleuchtungsmoduls gemäß Fig. 1 in ein Endoskop,
- Fig. 3: eine schematische Querschnittsansicht einer Anschlusslei- tung gemäß einer ersten Ausführungsform der Erfindung,
- Fig. 4: schematisch eine Querschnittsansicht einer Anschlusslei- tung gemäß einer zweiten Ausführungsform der Erfindung,
- Fig. 5: schematisch eine Querschnittsansicht gemäß einer dritten Ausführungsform der Erfindung,
- Fig. 6a: in einer distalseitigen Draufsicht ein erstes Beispiel für die Anordnung eines LED-Beleuchtungsmoduls in einem Endoskop,
- Fig. 6b: in einer perspektivischen Ansicht die Anordnung gemäß Fig. 6a,
- Fig. 7a: in einer distalseitigen Draufsicht ein zweites Beispiel für die Anordnung von LED-Beleuchtungsmodulen in einem Endoskop,
- Fig. 7b: in einer perspektivischen Ansicht die Anordnung gemäß Fig. 7a,
- Fig. 8: in einer distalseitigen Draufsicht ein drittes Beispiel für die Anordnung von LED-Beleuchtungsmodulen in einem Endoskop,
- Fig. 9: in einer distalseitigen Draufsicht ein viertes Beispiel für die Anordnung von LED-Beleuchtungsmodulen in einem Endoskop,
- Fig. 10: in einer distalseitigen Draufsicht ein fünftes Beispiel für die Anordnung von LED-Beleuchtungsmodulen in einem Endoskop,
- Fig. 11: in einer teilweise geschnittenen Ansicht ein erstes Beispiel für die Anbindung zweier LED an eine Anschlussleitung, welches nicht der Erfindung entspricht,
- Fig. 12: in einer perspektivischen Ansicht ein zweites Beispiel für die Anbindung zweier LED an eine Anschlussleitung und
- Fig. 13: in einer perspektivischen Ansicht ein drittes Beispiel für die Anbindung zweier LED an eine Anschlussleitung.

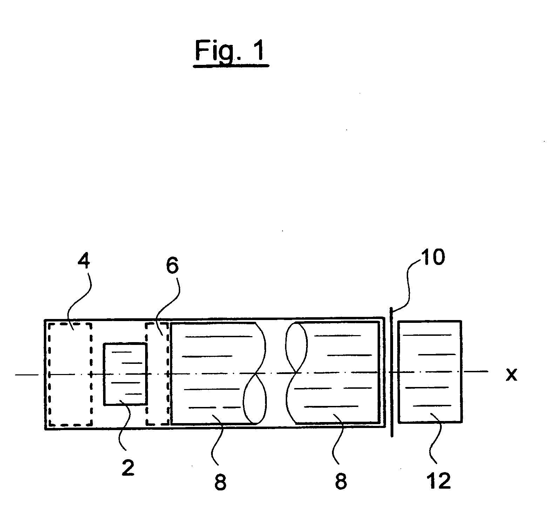

Wie schematisch in Fig. 1 gezeigt, weist ein erfindungsgemäßes Beleuchtungsmodul eine LED-Einheit 2 auf, welche im Bereich des distalen Endes angeordnet ist. Die LED-Einheit kann ein oder mehrere LED beinhalten. Distalseitig der LED-Einheit 2 ist diese im hier gezeigten Beispiel von einer Schutzglaseinheit 4 abgedeckt. Diese Schutzglaseinheit 4, die nicht unbedingt Teil des LED-Beleuchtungsmoduls sein muss und daher gestrichelt dargestellt ist, sondern auch dem distalen Ende des Endoskops zugeordnet sein kann, kann auch als Linse oder Linseneinheit ausgebildet sein. Diese Schutzglaseinheit 4 verschließt das Beleuchtungsmodul nach außen und schützt die proximalseitig dahinter liegende LED-Einheit 2 vor Verschmutzung und Beschädigung. Darüber hinaus kann die Schutzglaseinheit 4 thermisch isolierend wirken, so dass von der LED-Einheit abgegebene Abwärme nicht oder nur in geringerem Umfang zum distalen Ende des LED-Beleuchtungsmoduls bzw. des Endoskops gelangt. Proximalseitig der LED-Einheit 2 ist beispielsweise wie hier gezeigt, aber nicht obligatorisch eine dünne Verbindungsplatte 6 angeordnet, über welche die LED-Einheit 2 elektrisch leitend mit einer Anschlussleitung 8, welche eine Verbindungseinheit bildet, verbunden ist. Diese Verbindungsplatte kann als Platine oder z. B. auch als metallisches Plättchen, Kupferplättchen o. ä. ausgebildet sein. Die Anordnung der LED-Einheit 2 an der Verbindungsplatte 6 ist so ausgebildet, dass nicht nur eine elektrische Kontaktierung, sondern auch eine hochwärmeleitende Verbindung zwischen der LED-Einheit 2 und der Anschlussleitung 8 geschaffen wird. Die hochwärmeleitende Verbindung zwischen der LED-Einheit 2 und der Anschlussleitung 8 kann entweder durch unmittelbare Anlage oder über eine wärmeleitende Verbindung, welche beispielsweise durch Löten, Schweißen oder Kleben erzeugt werden kann.

Am distalen Ende der Anschlussleitung 8 ist eine Schnittstelle 10 vorgesehen, welche beispielsweise am proximalen Ende eines Endoskopschaftes angeordnet sein kann. Diese Schnittstelle 10 dient zum einen der elektrischen Kontaktierung der Anschlussleitung 8 und zum anderen auch der thermischen Kontaktierung. Beispielsweise kann diese Schnittstelle 10 wiederum die Verbindung zu einer Platine 12, welche zum elektrischen Anschluss dient, herstellen.

Erfindungsgemäß ist vorgesehen, dass die von der LED-Einheit 2 erzeugte Abwärme direkt auf die Anschlussleitung 8 übertragen wird und von dieser proximalwärts geleitet wird und bereits ein wesentlicher Teil dieser Abwärme über den Mantel der Anschlussleitung 8 an den Endoskopschaft und von dort an die Umgebung abgegeben wird. Der gegebenenfalls am proximalen Ende der Anschlussleitung 8 noch vorhandene Restanteil an Abwärme wird über die proximalseitige Schnittstelle 10 an weitere wärmeabführende Elemente oder die Umgebung abgegeben. Bei vorzugsweise gleichzeitiger thermischer Isolierung der LED-Einheit in lateraler und distaler Richtung (dort beispielsweise über die Schutzglaseinheit 4) kann so erreicht werden, dass von der LED-Einheit 2 erzeugte Abwärme zumindest nicht vollständig direkt an die Umgebung der LED-Einheit abgegeben wird, sondern von dieser gezielt in proximaler Richtung über die Anschlussleitung 8 abgeführt wird. Dazu werden insbesondere die elektrischen Leiter der Anschlussleitung 8, welche aus Metall ausgebildet sind, in ihrem Querschnitt so dimensioniert, dass sie eine ausreichende Wärmeleitzahl aufweisen, um die anfallende Verlustwärme vollständig oder zumindest zum großen Teil abzuführen. Insofern werden übermäßige Erwärmungen und ggf. damit verbundene Verletzungen, beispielsweise vom umgebenden Gewebe in Umgebung der LED-Einheit 2 verhindert.

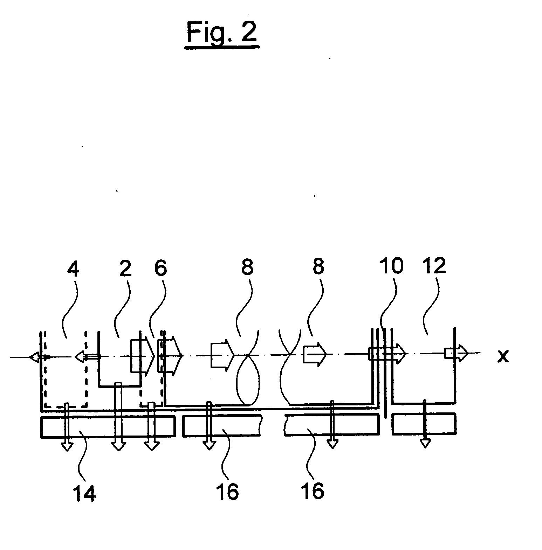

Fig. 2 zeigt nun schematisch die Einbindung des LED-Beleuchtungsmoduls gemäß Fig. 1 in ein Endoskop. Wie dort zu erkennen, sind beispielsweise die LED-Einheit 2, die optionale Verbindungsplatte 6 und die die LED-Einheit 2 distalseitig abdeckende Schutzglaseinheit 4, die je nach Ausführungsform dem LED-Beleuchtungsmodul oder dem distalen Ende des Endoskops zugeordnet und daher in der Fig. 2 gestrichelt dargestellt ist, in einem Endoskopkopf 14 angeordnet. Dieser kann beispielsweise starr ausgebildet sein, während ein sich in Richtung der Längsachse X proximalseitig anschließender Endoskopschaft 16 beispielsweise flexibel ausgebildet sein kann. Es können jedoch auch Endoskopkopf 14 und Endoskopschaft 16 starr oder vollständig flexibel ausgebildet sein. Die Anschlussleitung 8 erstreckt sich durch den Endoskopschaft 16 zum proximalen Ende des Endoskopes hin, an welchem die Schnittstelle 10 zur elektrischen Kontaktierung einer proximalseitigen Platine 12 angeordnet ist. Die Schnittstelle 10 muss nicht unbedingt zur Kontaktierung der Platine 12 dienen. Hier kann beispielsweise auch ein Anschlussstecker oder Ähnliches zur elektrischen Kontaktierung vorgesehen sein. Gegebenenfalls kann sich die Anschlussleitung 8 aber auch über das Endoskop hinaus bis zu einem externen Gerät erstrecken.

Durch die in Fig. 2 dargestellten Pfeile wird der erfindungsgemäß erzielte Wärmefluss im Endoskop schematisch dargestellt. Wie durch die dünnen Pfeile dargestellt wird nur ein geringer Teil der Wärme von der LED-Einheit 2 in die unmittelbare Umgebung, d. h. über die Wandung des Endoskopkopfes 14 und die Schutzglaseinheit 4 abgeleitet. Der größte Teil der Wärme wird, wie durch die breiteren Pfeile dargestellt, in proximaler Richtung über eine hochwärmeleitende Verbindung auf direkt von der LED-Einheit 2 auf die Anschlussleitung 8 übertragen. Von der Anschlussleitung 8 wird die Wärme weiter proximalwärts geführt, wobei die Anschlussleitung 8 abhängig vom Aufbau des Endoskops einen Teil der Wärme oder auch die gesamte Wärme in lateraler Richtung abgibt, wodurch sich die weiter proximalwärts zu übertragende Wärmemenge mit zunehmender Entfernung vom distalen Ende des Endoskops verringert. So wird nur noch ein Teil der von der LED-Einheit 2 auf die Anschlussleitung 8 übertragenen Abwärme, gegebenenfalls überhaupt keine Wärme mehr von der Anschlussleitung 8 proximalseitig über die Schnittstelle 10 abgeführt. Ein weiterer Teil der abgeführten Wärme wird über die Anschlussleitung 8 im Verlauf ihrer Erstreckung in Längsrichtung X an die Umgebung abgegeben. Erfindungsgemäß wird in diesem Beispiel somit erreicht, dass die von der LED-Einheit 2 erzeugte Abwärme nicht direkt in die unmittelbare Umgebung abgegeben wird, sondern in proximaler Richtung abgegeben wird und dort über die Anschlussleitung 8 über eine größere Fläche an die Umgebung abgegeben wird, wodurch punktuelle Temperaturspitzen, welche zu Verbrennungen beispielsweise umgebenden Gewebes führen könnten, vermieden werden.

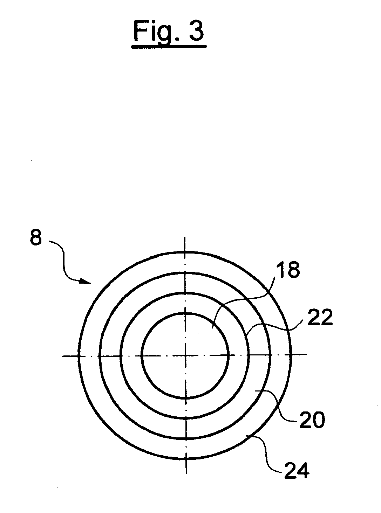

Die Anschlussleitung 8 ist erfindungsgemäß als Koaxialkabel ausgebildet. Beispiele hierfür sind in den Figuren 3 bis 5 gezeigt. Fig. 3 zeigt eine erste Ausführungsform eines solchen Koaxialkabels mit einem inneren Leiter 18 und einem äußeren, den inneren Leiter 18 konzentrisch umgebenden, ringförmigen Leiter 20. Der innere Leiter 18 und der äußere Leiter 20 sind durch eine ringförmige Isolationsschicht 22 elektrisch voneinander isoliert. Zusätzlich ist in dem Ausführungsbeispiel gemäß Fig. 3 eine äußere Isolationsschicht 24 vorgesehen, welche den äußeren Leiter 20 umfänglich umgibt. Erfindungsgemäß ist vorgesehen, dass sowohl der innere Leiter 18 als auch der äußere Leiter 20 zum elektrischen Anschluss der LED-Einheit 2 dienen. Gleichzeitig dienen die Leiter 18 und 20 zur Wärmeabfuhr in proximaler Richtung, wie anhand von Figuren 1 und 2 erläutert. Hierzu weist zumindest einer der Leiter eine Querschnittsfläche auf, welche größer, vorzugsweise mehr als drei- oder fünfmal größer als diejenige Querschnittsfläche ist, welche zur Übertragung der erforderlichen elektrischen Energie zur der LED-Einheit 2 erforderlich wäre. Auf diese Weise wird ein wesentlich größerer absoluter Wärmeleitwert erreicht, als sie bei herkömmlichen elektrischen Anschlussleitungen, wie sie zum Anschluss der verwendeten LED-Einheit erforderlich wären, Verwendung finden würde.

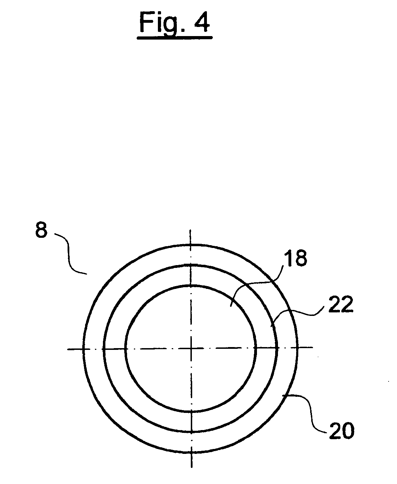

Um den Wärmeleitwert der Anschlussleitung 8 bzw. deren elektrischer Leiter 18 und 20 zu erhöhen, ist es erstrebenswert, die Querschnittsflächen der elektrischen Leiter 18 und 20 zu optimieren. Hierzu ist ein weiteres Ausführungsbeispiel schematisch in Fig. 4 gezeigt. Bei diesem Beispiel ist auf die äußere Isolationsschicht 24 verzichtet. Dies ermöglicht es, bei gleichem Gesamtdurchmesser der Anschlussleitung 8 im Vergleich zu dem Ausführungsbeispiel in Fig. 3 die Querschnittsflächen der elektrischen Leiter 18 und 20 zu vergrößern, indem der Durchmesser des inneren Leiters 18 vergrößert und/oder die radiale Dicke des äußeren Leiters 20 vergrößert werden kann.

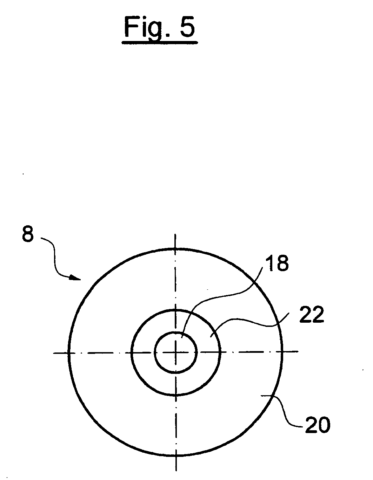

Eine weitere Optimierung des Querschnittes der elektrischen Leiter 18 und 20 ist in dem Ausführungsbeispiel gemäß Fig. 5 zu erkennen. Dort ist ebenfalls auf die äußere Isolationsschicht 24 (siehe Fig. 3) verzichtet. Darüber hinaus ist jedoch der innere Leiter 18 in seinem Durchmesser minimiert. Idealerweise hat der innere Leiter 18 dabei nur einen solchen Durchmesser bzw. eine solche Querschnittsfläche, welche zur Übertragung der geforderten elektrischen Leistung bzw. des auftretenden elektrischen Stromes erforderlich ist. Dies führt zu einem im Durchmesser vergleichsweise kleinen inneren Leiter 18, wodurch die Umfangslänge der erforderlichen Isolationsschicht 22, welche den inneren Leiter 18 am Außenumfang umgibt, verkleinert wird. Dadurch verkleinert sich die Gesamtquerschnittsfläche der Isolationsschicht 22, und der äußere Leiter 20 kann in radialer Richtung besonders dick ausgebildet werden, so dass insgesamt die Querschnittsfläche der elektrischen Leiter 18 und 20 zusammen bei konstantem Außendurchmesser maximiert werden kann. Prinzipiell kann, um eine hohe Flexibilität der Anschlussleitung 8 zu gewährleisten, der Leiter 20 auch aus mehreren Schichten, die sich in longitudinaler Richtung gegeneinander verschieben lassen, bestehen. Mit der größeren Querschnittsfläche der metallischen Leiter 18 und 20 verbessert sich auch die Wärmeleitfähigkeit bzw. es erhöht sich die absolute Wärmeleitzahl, wodurch eine bessere Wärmeabfuhr von der LED-Einheit 2 erreicht wird.

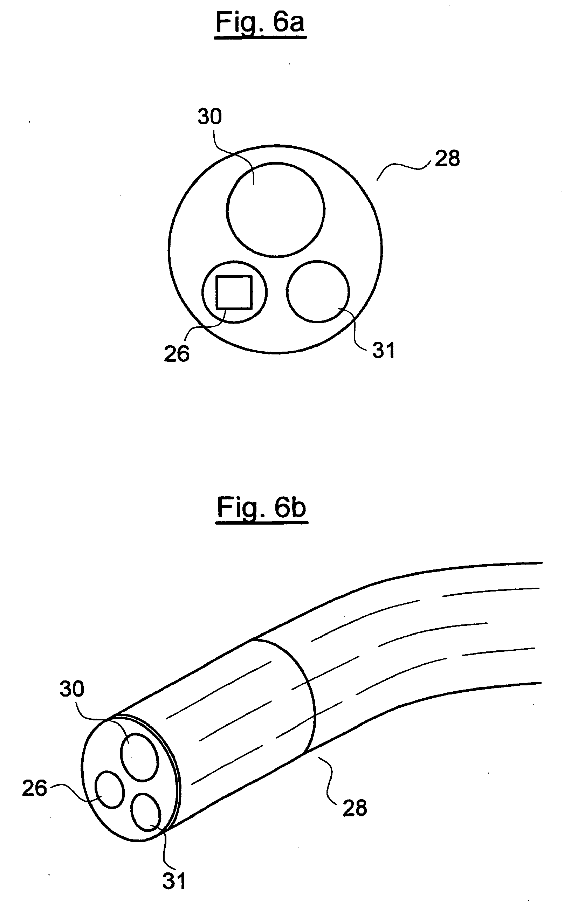

Anhand der Figuren 6 bis 10 wird nun nachfolgend beispielhaft die Anordnung des zuvor beschriebenen LED-Beleuchtungsmoduls bzw. mehrerer LED-Beleuchtungsmodule in einem Endoskop beschrieben. Dazu zeigen die Figuren 6 bis 8 schematisch distale Draufsichten und Schrägansichten auf einen Endoskopschaft.

Bei dem Ausführungsbeispiel gemäß Fig. 6a und 6b ist in dem Endoskopschaft ein LED-Beleuchtungsmodul 26 vorgesehen, welches bevorzugt in der vorangehend beschriebenen Weise ausgestaltet sein kann. Da der Endoskopschaft 28 bei diesem Ausführungsbeispiel vergleichsweise dünn, beispielsweise flexibel ausgestaltet ist, ist in dem LED-Beleuchtungsmodul 26 nur eine LED vorgesehen. Darüber hinaus sind in dem Endoskopschaft 28 noch ein Instrumentenkanal 30 und eine Bildsensoreinheit 31 angeordnet.

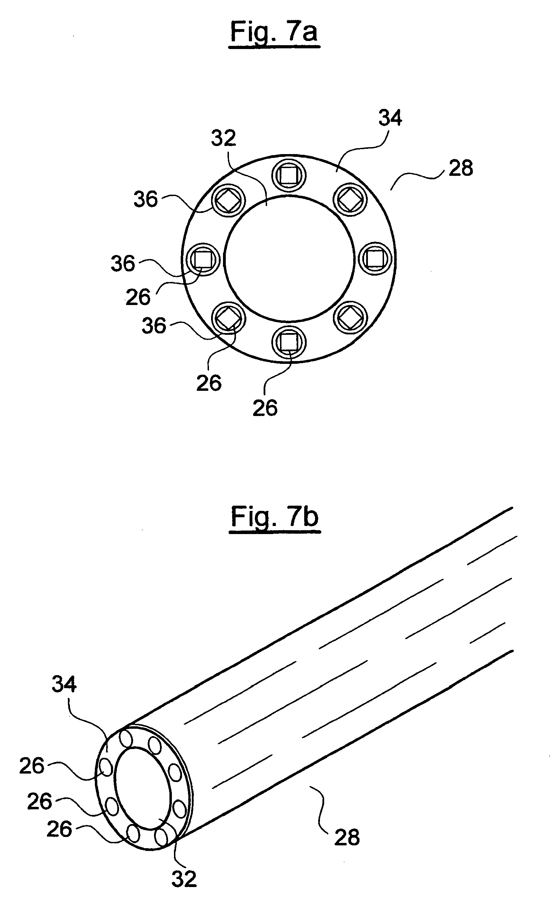

Bei dem Ausführungsbeispiel gemäß Fig. 7a und 7b sind mehrere, hier acht LED-Beleuchtungsmodule 26 vorgesehen, welche ringförmig um einen zentralen Kanal angeordnet sind. Auch hier können die Beleuchtungsmodule 26 in der zuvor beschriebenen Weise ausgestaltet sein. Dabei können die mehreren einzelnen LED-Beleuchtungsmodule 26 jeweils eine eigene Anschlussleitung 8 aufweisen, welche sich proximalseitig erstreckt. Um die Wärmeflussdichte in radialer Richtung von den LED-Beleuchtungsmodulen 26 zu dem Schaftrohr 34 möglichst gering zu halten, sind die einzelnen Beleuchtungsmodule 26 jeweils von einem dünnen Kunststoffschlauch 36 oder einem Schlauch aus einem anderen Material mit einer vergleichsweise geringen Wärmeleitfähigkeit umgeben, womit eine Reduzierung des Wärmeflusses in lateraler Richtung erzielt wird. Dies ist insbesondere deshalb von Vorteil, da bei diesem Ausführungsbeispiel die LED-Beleuchtungsmodule 26 sehr nahe zu dem umgebenden Schaftrohr 34 angeordnet sind, welches bei einem starren Endoskop zudem aus Metall ausgebildet ist. Insofern kann durch die Schläuche 36 aus einem Material mit einer vergleichsweise geringen Wärmeleitfähigkeit eine übermäßige Wärmeübertragung auf das Schaftrohr 34 verhindert werden und die Wärme gezielt, wie oben beschrieben, über die Anschlussleitungen 8 proximalwärts abgeführt und kontinuierlich über eine große Länge/ eine große Mantelfläche des Schaftrohrs 34 an die Umgebung abgegeben werden.

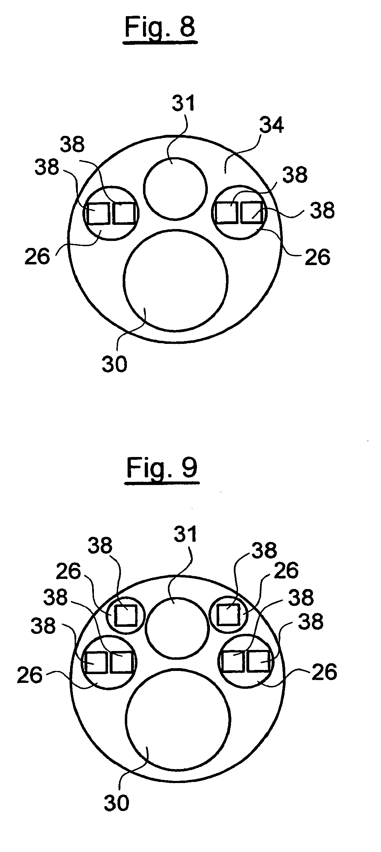

Bei dem Ausführungsbeispiel gemäß Fig. 8 sind in dem Schaft 34 oder Endoskopkopf zwei Beleuchtungseinheiten 26 seitlich einer Bildsensoreinheit 31 angeordnet. Die LED-Beleuchtungsmodule 26 weisen dabei im hier gezeigten Beispiel jeweils zwei LED 38 auf. Zusätzlich ist in dem Schaft 34 oder Endoskopkopf noch ein Instrumentenkanal 30 vorgesehen. Bei dem Ausführungsbeispiel gemäß Fig. 9 sind vier LED-Beleuchtungsmodule 26 vorgesehen, zwei mit jeweils zwei LED 38 und zwei mit jeweils einer LED 38, welche umfänglich einer Bildsensoreinheit 31 angeordnet sind. Bei den mehreren LED-Beleuchtungsmodulen 26 kann es sich beispielsweise um LED-Beleuchtungsmodule 26 handeln, welche unterschiedliche Wellenlängen emittieren. So können beispielsweise zwei LED-Beleuchtungsmodule 26, beispielsweise diejenigen, welche nur eine LED 38 aufweisen, zur Abgabe von Weißlicht und die beiden anderen LED-Beleuchtungsmodule 26 zur Abgabe von violettem Anregungslicht zur Fluoreszenz-Endoskopie ausgebildet sein.

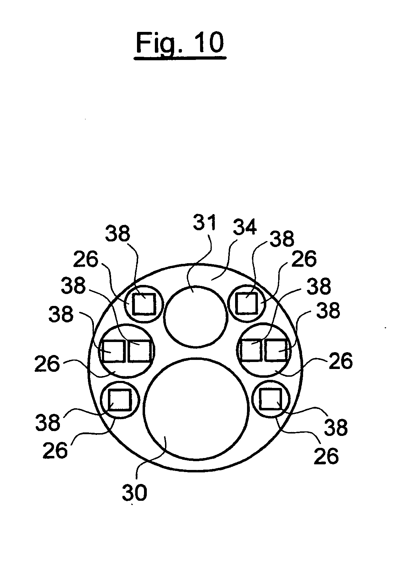

Fig. 10 zeigt eine Anordnung von insgesamt sechs LED-Beleuchtungsmodulen 26 in einem Schaftrohr 34. Auch hier können die verschiedenen LED-Beleuchtungsmodule zur Abgabe von Licht von unterschiedlichen Wellenlängen ausgebildet sein. Auch hier sind die Bildsensoreinheit 31 und Instrumentenkanal 30 in der voranbeschriebenen Weise vorgesehen. An diesem Beispiel wird deutlich, wie durch die Verfügbarkeit und den Einsatz von Beleuchtungsmodulen unterschiedlicher Größe und mit gegebenenfalls unterschiedlichen Funktionen (z. B. unterschiedliche Emissionsbereiche) der verfügbare Raum in optimaler Weise genutzt werden kann, wie also unter Nutzung des modularen Konzepts ohne eine Vergrößerung des Gesamtdurchmessers des Endoskops die bereitgestellte Lichtmenge und/oder die Funktionalität optimiert werden kann.

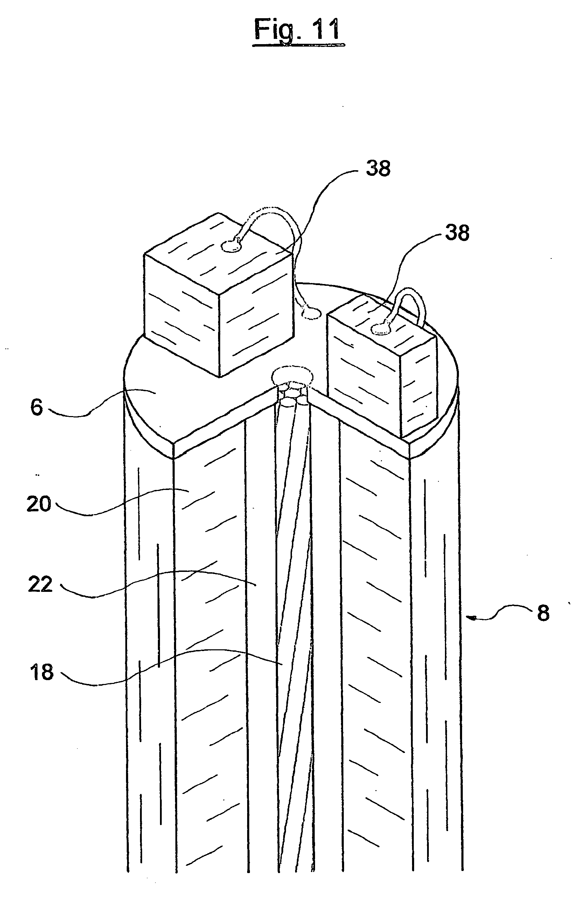

Die Figuren 11 bis 13 zeigen drei Beispiele der Anbindung von LED 38, welche LED-Einheiten 2 bilden, an eine Anschlussleitung 8. Die Anschlussleitung 8 in Form eines Koaxialkabels, welche in Fig. 11 gezeigt ist, weist wie vorangehend beschrieben einen inneren und einen äußeren Leiter 18 und 20 mit einer dazwischenliegenden Isolationsschicht 22 auf. Das distalseitige Ende der Anschlussleitung 8 stößt flächig an eine Verbindungsplatte 6 an, so dass hier eine hochwärmeleitende, großflächige Verbindung zwischen der Stirnseite der Anschlussleitung 8 und der Verbindungsplatte 6 geschaffen wird. D. h. hier ist entgegen der Erfindung keine direkte Verbindung zwischen LED und Koaxialkabel gegeben. Es kann beispielsweise eine wärmeleitende Verklebung oder Lötverbindung vorgesehen sein. Handelt es sich bei der Verbindungsplatte 6 um eine Platine ist diese im Sinne eines geringen thermischen Widerstandes und einer guten Wärmeübertragung in longitudinaler Richtung X sehr dünn ausgeführt. Für eine weitere Verringerung des thermischen Widerstandes kann sie mit Um-, Durch- oder Ankontaktierungen versehen sein. An der Verbindungsplatte 6 sind zwei LED 38, welche die LED-Einheit 2 bilden, elektrisch leitend und wärmeleitend angebracht. So wird auch hier zwischen den LED 38 und der Verbindungsplatte 6 in geeigneter Weise eine hochwärmeleitende Verbindung geschaffen, so dass die Abwärme der LED 38 über die Verbindungsplatte 6 auf die metallischen Leiter 18 und 20 der Anschlussleitung 8 übertragen und von dieser proximalwärts abgeführt werden kann.

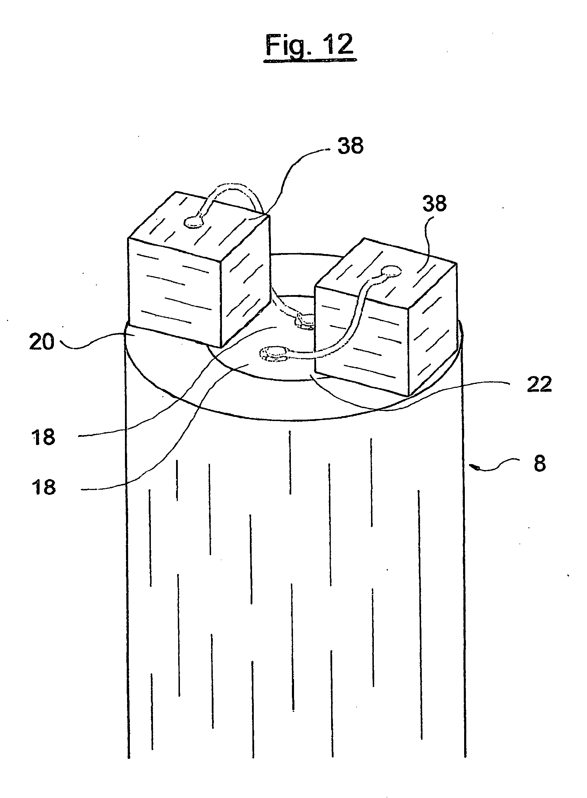

Fig. 12 zeigt eine Ausführungsform, bei welcher die die LED-Einheit 2 bildenden LED 38 direkt an die Stirnseite der Anschlussleitung 8 angesetzt sind. Bei dem Ausführungsbeispiel gemäß Fig. 12 sind zwei Innenleiter 18 vorgesehen, welche innerhalb einer Isolationsschicht 22 gegeneinander isoliert angeordnet sind. Auf diese Weise können die zwei LED 38 unabhängig voneinander mit elektrischer Energie versorgt werden. So können innerhalb eines einzigen Beleuchtungsmoduls die LED 38 unterschiedlich angesteuert werden. Damit wird es beispielsweise möglich, den LED 38 unterschiedliche Funktionen zukommen zu lassen und diese anwendungsorientiert einzusetzen (z. B. Weißlichtbeleuchtung mit weißer LED und andererseits Fluoreszenzanregung mit blauer LED. Die LED 38 sind so angeordnet, dass sie großflächig direkt an der Stirnseite des äußeren Leiters 20 anliegen, welcher der Wärmeabfuhr dient. Dazu ist eine hochwärmeleitende Verbindung zwischen den LED 38 und dem äußeren Leiter 20 beispielsweise durch Verlöten oder Verkleben vorgesehen. Die inneren Leiter 18 werden beispielsweise mit Golddrähten an die entsprechende Anschlussfläche der LED 38 angebondet.

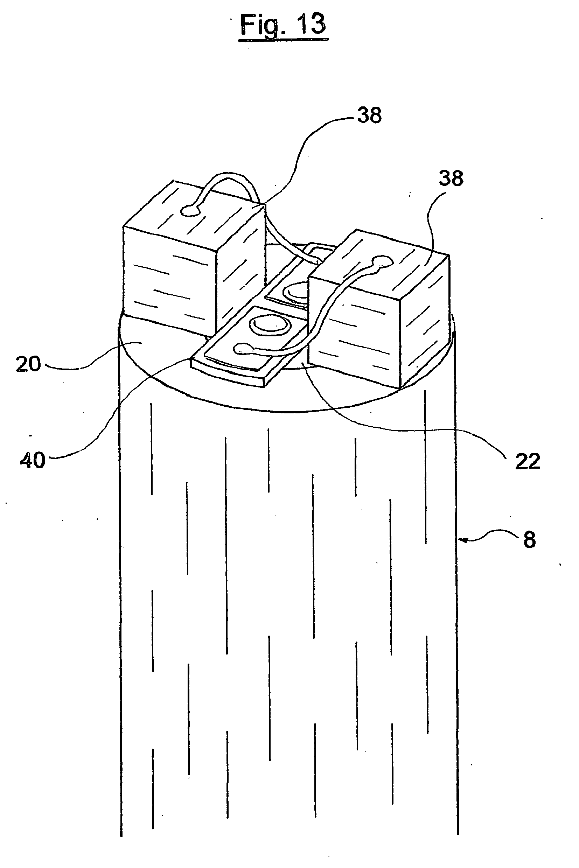

Die Ausführungsform gemäß Fig. 13 entspricht der Ausführungsform gemäß Fig. 12, wobei noch eine Platine 40 zwischen den LED 38 angeordnet ist, welche der elektrischen Kontaktierung der beiden LED 38 mit den beiden Innenleitern 18 (siehe Fig. 12) dient.

Die erfindungsgemäßen LED-Beleuchtungsmodule können in unterschiedlicher Form realisiert werden. Entsprechend den Anforderungen (Lichtmenge, Wellenlänge) und den geometrischen Verhältnissen im Endoskop können dann passende LED-Beleuchtungsmodule eingesetzt werden.

### Bezugszeichenliste

- 2: - LED-Einheit
- 4: - Schutzglaseinheit
- 6: - Verbindungsplatte
- 8: - Anschlussleitung
- 10: - Schnittstelle
- 12: - Platine
- 14: - Endoskopkopf
- 16: - Endoskopschaft
- 18: - innerer Leiter
- 20: - äußerer Leiter
- 22, 24: - Isolationsschichten
- 26: - LED-Beleuchtungsmodul
- 28: - Endoskopschaft
- 30: - Instrumentenkanal
- 31: - Bildsensoreinheit
- 32: - zentraler Kanal
- 34: - Schaftrohr
- 36: - Kunststoffschlauch
- 38: - LED
- 40: - Platine
- X: - Längsachse

## Patentansprüche

1. Endoskopisches Instrument mit zumindest einem LED-Beleuchtungsmodul mit zumindest einer am distalen Ende des Instrumentes angeordneten LED (38) und einer an dieser angebrachten elektrischen Anschlussleitung (8), wobei
die Anschlussleitung (8) ein Koaxialkabel ist, welches sich vom distalen Ende zum proximalen Ende des Instrumentes erstreckt und zur Abfuhr der von der LED (38) erzeugten Abwärme ausgebildet ist, **dadurch gekennzeichnet, dass** zumindest ein elektrischer Leiter (18, 20) des Koaxialkabels direkt wärmeleitend mit der LED (38) verbunden ist.

2. Endoskopisches Instrument nach Anspruch 1, **dadurch gekennzeichnet, dass** mehrere elektrische Leiter (18, 20) der Anschlussleitung (8) wärmeleitend mit der LED (38) verbunden sind.

3. Endoskopisches Instrument nach Anspruch 1 oder 2, **dadurch gekennzeichnet, dass** der zumindest eine elektrische Leiter (18, 20) der Anschlussleitung eine größere, vorzugsweise mindestens dreimal größere Querschnittsfläche aufweist, als für die elektrische Leistungsübertragung zu der LED (38) erforderlich ist.

4. Endoskopisches Instrument nach einem der Ansprüche 1 bis 3, **dadurch gekennzeichnet, dass** der zumindest eine elektrische Leiter (18, 20) des Anschlusskabels (8) mit einem Maximum seiner Querschnittsfläche wärmeleitend mit der LED (38) verbunden ist.

5. Endoskopisches Instrument nach einem der vorangehenden Ansprüche, **dadurch gekennzeichnet, dass** die elektrische Anschlussleitung (8) an einer rückseitigen Fläche wärmeleitend mit der LED (38) verbunden ist.

6. Endoskopisches Instrument nach einem der vorangehenden Ansprüche, **dadurch gekennzeichnet, dass** zumindest der äußere elektrische Leiter (20) der Anschlussleitung (8) wärmeleitend mit der LED (38) verbunden ist.

7. Endoskopisches Instrument nach einem der vorangehenden Ansprüche, **dadurch gekennzeichnet, dass** der äußere Leiter (20) des Koaxialkabels in radialer Richtung eine größere Dicke als eine Isolationsschicht (22) aufweist, welche zwischen einem inneren (18) und dem äußeren (20) elektrischen Leiter gelegen ist.

8. Endoskopisches Instrument nach einem der vorangehenden Ansprüche, **dadurch gekennzeichnet, dass** der innere Leiter (18) des Koaxialkabels eine minimierte Querschnittsfläche aufweist, welche in ihrer Größe an die zu übertragende elektrische Leistung angepasst ist.

9. Endoskopisches Instrument nach einem der vorangehenden Ansprüche, **dadurch gekennzeichnet, dass** der äußere Leiter (20) des Koaxialkabels an seinem Außenumfang keine elektrische Isolation aufweist.

10. Endoskopisches Instrument nach einem der vorangehenden Ansprüche, **dadurch gekennzeichnet, dass** die die LED (38) umfänglich und/oder frontseitig von Elementen (4, 36) umgeben ist, welche eine geringere Wärmeleitfähigkeit als die elektrische Anschlussleitung (8) aufweisen.

11. Endoskopisches Instrument nach einem der vorangehenden Ansprüche, **dadurch gekennzeichnet, dass** die Anschlussleitung (8) flexibel ausgebildet ist.

12. Endoskopisches Instrument nach einem der vorangehenden Ansprüche, **dadurch gekennzeichnet, dass** zumindest einer der Leiter der Anschlussleitung aus mehreren Einzelleitern, insbesondere aus einer Vielzahl von Litzen gebildet ist.

13. Endoskopisches Instrument nach einem der vorangehenden Ansprüche, **dadurch gekennzeichnet, dass** das LED-Beleuchtungsmodul austauschbar ausgebildet ist.

## Claims

1. An endoscopic instrument with at least one LED illumination module with at least one LED (38) arranged at the distal end of the instrument and with an electrical connection lead (8) attached to this LED, wherein the connection lead (8) is a coaxial cable which extends from the distal end to the proximal end of the instrument and is designed for leading away the waste heat produced by the LED (38), **characterised in that** at least one electrical conductor (18, 20) of the coaxial cable is directly connected to the LED (38) in a heat-conducting manner.

2. An endoscopic instrument according to claim 1, **characterised in that** several electrical conductors (18, 20) of the connection lead (8) are connected to the LED (38) in a heat-conducting manner.

3. An endoscopic instrument according to claim 1 or 2, **characterised in that** the at least one electrical conductor (18, 20) of the connection lead has a larger, preferably at least three times larger cross-sectional area, than is necessary for the electrical power transmission to the LED (38).

4. An endoscopic instrument according to one of the claims 1 to 3, **characterised in that** the at least one electrical conductor (18, 20) of the connection cable (8) is connected in a heat-conducting manner with a maximum of its cross-sectional area, to the LED (38).

5. An endoscopic instrument according to one of the preceding claims, **characterised in that** the electrical connection lead (8) at a rear-side surface is connected in a heat-conducting manner to the LED (38).

6. An endoscopic instrument according to one of the preceding claims, **characterised in that** at least the outer electrical conductor (20) of the connection lead (8) is connected to the LED (38) in a heat-conducting manner.

7. An endoscopic instrument according to one of the preceding claims, **characterised in that** the outer conductor (20) of the coaxial cable has a greater thickness in the radial direction than an insulation layer (22) which is situated between an inner (18) and the outer (20) electrical conductor.

8. An endoscopic instrument according to one of the preceding claims, **characterised in that** the inner conductor (18) of the coaxial cable has a minimized cross-sectional area which in its size is adapted to the electrical power to be transmitted.

9. An endoscopic instrument according to one of the preceding claims, **characterised in that** the outer conductor (20) of the coaxial cable has no electrical insulation at its outer periphery.

10. An endoscopic instrument according to one of the preceding claims, **characterised in that** the LED (38) is surrounded peripherally and/or at the front side, by elements (4, 36) which have a lower thermal conductivity than the electrical connection lead (8).

11. An endoscopic instrument according to one of the preceding claims, **characterised in that** the connection lead (8) is designed in a flexible manner.

12. An endoscopic instrument according to one of the preceding claims, **characterised in that** at least one of the conductors of the connection lead is formed of several individuals conductors, in particular of a multitude of strands.

13. An endoscopic instrument according to one of the preceding claims, **characterised in that** the LED illumination module is designed in an exchangeable manner.

## Revendications

1. Instrument endoscopique, comprenant au moins un module d'éclairage par LED doté d'au moins une LED (38) disposée à l'extrémité distale de l'instrument et d'un câble de raccordement électrique (8) monté sur celle-ci,
le câble de raccordement (8) étant un câble coaxial qui s'étend de l'extrémité distale à l'extrémité proximale de l'instrument et qui est conçu pour évacuer la chaleur dégagée par la LED (38), **caractérisé en ce qu'**au moins un conducteur électrique (18, 20) du câble coaxial est directement relié à la LED (38) de manière thermoconductrice.

2. Instrument endoscopique selon la revendication 1, **caractérisé en ce que** plusieurs conducteurs électriques (18, 20) du câble de raccordement (8) sont reliés à la LED (38) de manière thermoconductrice.

3. Instrument endoscopique selon la revendication 1 ou 2, **caractérisé en ce que** ledit au moins un conducteur électrique (18, 20) du câble de raccordement présente une superficie de section transversale plus grande, de préférence, au moins trois fois plus grande que celle nécessaire pour transmettre la puissance électrique à la LED (38).

4. Instrument endoscopique selon l'une des revendications 1 à 3, **caractérisé en ce que** le au moins un conducteur électrique (18, 20) du câble de raccordement (8) est relié à la LED (38) de manière thermoconductrice avec un maximum de sa superficie de section transversale.

5. Instrument endoscopique selon l'une des revendications précédentes, **caractérisé en ce que** le câble de raccordement électrique (8) est relié de manière thermoconductrice à la LED (38) au niveau d'une face arrière.

6. Instrument endoscopique selon l'une des revendications précédentes, **caractérisé en ce qu'**au moins le conducteur électrique extérieur (20) du câble de raccordement (8) est relié de manière thermoconductrice à la LED (38).

7. Instrument endoscopique selon l'une des revendications précédentes, **caractérisé en ce que** le conducteur extérieur (20) du câble coaxial présente, en direction radiale, une épaisseur plus grande qu'une couche d'isolation (22) interposée entre un conducteur électrique intérieur (18) et le conducteur électrique extérieur (20).

8. Instrument endoscopique selon l'une des revendications précédentes, **caractérisé en ce que** le conducteur intérieur (18) du câble coaxial présente une superficie de section transversale réduite dont la dimension est adaptée à la puissance électrique à transmettre.

9. Instrument endoscopique selon l'une des revendications précédentes, **caractérisé en ce que** le conducteur extérieur (20) du câble coaxial ne présente aucune isolation électrique sur sa périphérie extérieure.

10. Instrument endoscopique selon l'une des revendications précédentes, **caractérisé en ce que** la LED (38) est entourée, sur sa périphérie et/ou sur son côté frontal, d'éléments (4, 36) qui présentent une conductivité thermique plus faible que le câble de raccordement électrique (8).

11. Instrument endoscopique selon l'une des revendications précédentes, **caractérisé en ce que** le câble de raccordement (8) est flexible.

12. Instrument endoscopique selon l'une des revendications précédentes, **caractérisé en ce qu'**au moins l'un des conducteurs du câble de raccordement est formé de plusieurs conducteurs individuels, notamment d'une pluralité de torons.

13. Instrument endoscopique selon l'une des revendications précédentes, **caractérisé en ce que** le module d'éclairage par LED est conçu de manière remplaçable.
